# EUROPÄISCHE PATENTSCHRIFT

(11) **EP 1 135 356 B1**
(45) Veröffentlichungstag und Bekanntmachung des Hinweises auf die Patenterteilung: **22.07.2009**
(21) Anmeldenummer: 99959316.3
(22) Anmeldetag: 24.11.1999
(51) Int. Cl.: C07C 51/58, C01B 17/45, C07C 53/18, C07C 211/03

(54) **VERFAHREN ZUR HERSTELLUNG VON SÄUREFLUORIDEN AUS SÄURECHLORIDEN**
METHOD OF PRODUCING ACID FLUORIDES FROM ACID CHLORIDES
PROCEDE POUR PREPARER DES FLUORURES D'ACIDE A PARTIR DE CHLORURES D'ACIDE

(30) Priorität: 30.11.1998 DE 19855252; 13.07.1999 DE 19932554; 04.09.1999 DE 19942374
(43) Veröffentlichungstag der Anmeldung: 26.09.2001
(60) Teilanmeldung: 05016647.9
(73) Patentinhaber: Solvay Fluor GmbH, 30173 Hannover (DE)
(72) Erfinder: BRAUN, Max, D-30900 Wedemark (DE); RIELAND, Matthias, D-30539 Hannover (DE); JANSSENS, Francine, B-1800 Vilvoorde (BE); EICHHOLZ, Kerstin, D-30855 Langenhagen (DE); PALSHERM, Stefan, D-30890 Barsinghausen (DE)
(74) Vertreter: Jacques, Philippe
(86) Internationale Anmeldenummer: PCT/EP1999/009060
(87) Internationale Veröffentlichungsnummer: WO 2000/032549

(56) Entgegenhaltungen:
- EP-A- 0 005 810
- US-A- 4 003 984
- FRANZ R: "Über Trishydrofluoride tertiärer Amine und ihren Einsatz als Fluorierungsmittel" JOURNAL OF FLUORINE CHEMISTRY, Bd. 15, Nr. 5, 1. Mai 1980 (1980-05-01), Seiten 423-34, XP000617966 ISSN: 0022-1139
- WEINLAND R F ET AL: "Über Hydrofluoride einiger, zum Teil sehr schwacher, organischer Basen" CHEMISCHE BERICHTE, Bd. 41, 1908, Seiten 3671-4, XP002132922
- NORRILD J C ET AL: "A facile and efficient synthesis of (+)- and (-)-allo-muscarine and analogs" SYNTHESIS, Nr. 10, Oktober 1997 (1997-10), Seiten 1128-30, XP002132923

## Beschreibung

Die Erfindung bezieht sich auf ein Verfahren zur Herstellung von Sulfurylfluorid aus Säurechlorid unter Kontaktieren mit Fluorwasserstoff-Addukten von Stickstoffbasen-Hydrofluoriden.

Säurefluoride, beispielsweise Carbonsäurefluoride und Sulfurylfluorid, sind interessante Produkte zur Verwendung per se oder als Zwischenprodukt. Sulfurylfluorid beispielsweise kann als Entwesungsmittel ("Fumigant") für Schädlinge, z. B. in verbautem Holz, Kirchen, Museen, Silos und Gebäuden und gegen verfärbende Enzyme, Pilze oder Krankheitserreger in nicht verbautem, z. B. frisch gefälltem Holz eingesetzt werden. Carbonsäurefluoride sind wertvolle Zwischenprodukte in der chemischen Synthese. Sulfurylchloridfluorid kann zu Sulfurylfluorid umgesetzt werden.

Die deutsche Offenlegungsschrift DE-OS 28 23 969 offenbart die Herstellung von Fluorcarbonyl-Verbindungen aus Chlorcarbonyl-Verbindungen, beispielsweise von Chlordifluoracetylfluorid aus Chlordifluoracetylchlorid und HF-Addukten von Hydrofluoriden organischer Stickstoffbasen als Fluorierungsmittel. Verwendet man HF-Addukte, wird Amin zur HF-Bindung zugesetzt oder es werden Lösungsmittel mit ausreichender Basizität zur HF-Bindung verwendet.

Das Journal of Fluorine Chemistry, Vol. 15 (1980), Seiten 423 bis 434 offenbart die Verwendung von Trishydrofluoriden tertiärer Amine als Fluorierungsmittel. Beispielsweise werden Fluoraceton, Cyanurfluorid, Difluorphosgen, Oxalylfluorid und Schwefeltetrafluorid hergestellt.

Die Europäische Patentanmeldung EP-A-0 358985 beschreibt eine Methode zur Herstellung von fluorierten Phosphorverbindungen durch den Austausch von Chlor, Brom oder Jod mittels eines Fluoridsalzes wie KF oder NH₄F in einem inerten Lösungsmittel in Anwesenheit einer katalytischen Menge einer Carbonsäure.

Die US-A-3 687626 offenbart, dass sich NH4HF2 sehr gut als Fluorierungsmittel bei der Herstellung von Sulfurylfluorid aus Sulfurylchlorid eignet.

Aufgabe der vorliegenden Erfindung war es, ein verbessertes Fluorierungsverfahren für die Herstellung von Sulfurylfluorid aus dem Säurechlorid anzugeben. Diese Aufgabe wird durch das Verfahren der vorliegenden Erfindung gelöst.

Das erfindungsgemäße Verfahren zur Herstellung von Sulfurylfluorid durch Kontaktieren von Säurechlorid und Fluorwasserstoff-Addukten von Hydrofluoriden organischer Stickstoffbasen ohne Zusatz einer HF-bindenden Base oder eines HF-bindenden Lösemittels sieht vor, dass das Fluorwasserstoff-Addukt als Fluorierungsreagenz dient und nicht über die Stufe des Hydrofluorids der organischen Stickstoffbase hinaus dehydrofluoriert wird.

Dabei arbeitet man, anders als im Stand der Technik, so, daß keine Base zugesetzt wird, die HF-bindend wirkt. Es wird auch kein HF-bindendes Lösungsmittel verwendet. Allenfalls, wenn man gemäß einer noch zu beschreibenden Ausführungsform eine Säure zusetzt, wie z. B. Trifluoressigsäure, kann man diese Säure oder einen Teil dieser Säure durch Base neutralisieren, indem man vor, während oder nach der Säurezugabe eine gewünschte Basenmenge zusetzt. HF wird aber nicht gebunden.

Zur Kontaktierung sind die in der deutschen Offenlegungsschrift DE-OS 28 23 969 genannten HF-Addukte von Hydrofluoriden organischer Stickstoffbasen brauchbar. Sie können durch die Formel B·(HF)ₓ ausgedrückt werden, darin stellt B eine organische Stickstoffbase dar und x bedeutet eine ganze oder gebrochene Zahl von >1 bis 4, vorzugsweise 2 - 3.

Als organische Stickstoffbasen B können alle möglichen primären, sekundären und/oder tertiären Amine einschließlich N-Heterozyklen verwendet werden. Wenn man als Formel für diese Amine angibt:

R₁R₂R₃N

so können darin bedeuten:
- R₁: einen Alkylrest, vorzugsweise mit 1 bis 10, insbesondere mit 1 bis 6 C-Atomen, einen Cycloalkylrest, vorzugsweise mit 5 bis 7 C-Atomen,
einen Aralkylrest, vorzugsweise mit 6 bis 10 C-Atomen oder
einen Arylrest, vorzugsweise ebenfalls mit 6 bis 10 C-Atomen;
- R₂ und R₃: Wasserstoff,
Alkyl-, Cycloalkyl-, Aralkyl- und Arylreste der gleichen Art wie bei R₁ angegeben.

Die Reste R₂ und R₃ können gleich oder verschieden sein. Zwei der Reste R₁ und R₂ oder R₃ können auch zu einem cycloaliphatischen Ring, welcher gegebenenfalls noch durch andere Heteroatome wie durch Sauerstoffatome unterbrochen sein kann, geschlossen sein. Ebenfalls ist es möglich, daß die drei Reste R₁, R₂ und R₃ Bestandteil eines heterocyclischen Ringes sind, wodurch dann entsprechende N-Heterocyclen resultieren. Bevorzugte organische Stickstoffbasen B sind primäre, sekundäre und/oder tertiäre Amine mit insgesamt bis zu 12 C-Atomen, wobei die sekundären und/oder tertiären aliphatischen Amine besonders bevorzugt sind.

Konkrete Beispiele für die Basen B sind:
N-Butylamin, N-Decylamin, Diäthylamin, Di-n-octylamin, Trimethylamin, Triäthylamin, Tri-n-propylamin, Isopropyldiäthylamin, Tri-n-butylamin, Cyclohexylamin, N-Methylanilin, N,N-Dimethylanilin, Pyrrolidin, Piperidin, N-Methylpiperidin, Morpholin, Pyridin, Chinolin etc.

Die HF-Addukte der Hydrofluoride der Stickstoffbasen B sind leicht aus den Basen B und Fluorwasserstoff erhältlich; sie sind niedrigschmelzende oder bei Raumtemperatur flüssige Substanzen mit beträchtlicher thermischer Belastbarkeit. Die Trishydrofluoride sind sogar unzersetzt vakuumdestillierbar.

Bevorzugt setzt man HF-Addukte von primären, sekundären oder tertiären Aminhydrofluoriden mit bis zu 15 C-Atomen ein, insbesondere von sekundären und tertiären Aminhydrofluoriden. Besonders gut geeignet sind HF-Addukte von Tri-n-propylaminhydrofluorid, Tri-iso-propylaminhydrofluorid, Tri-n-butylaminhydrofluorid, Pyridinhydrofluorid, Piperidinhydrofluorid oder N,N-Dimethylaminhydrofluorid.

Man führt die Umsetzung in flüssiger Phase durch.

Das erfindungsgemäße Verfahren kann batchweise oder kontinuierlich durchgeführt werden. Bei kontinuierlicher Fahrweise kann man so vorgehen, daß neben dem zu fluorierenden Säurechlorid auch HF, frisches HF-Addukt oder beides eingespeist wird. Entsprechend wird Reaktionsmischung abgetrennt bzw. gasförmige Reaktionsprodukte abdestilliert oder gasförmig abgeführt.

Die Erfinder haben festgestellt, daß sich im Laufe der Zeit HCl in der Reaktionsmischung anreichert. Wenn nicht intermittierend oder kontinuierlich HF eingespeist wird, verarmt die Reaktionsmischung bzw. das HF-Addukt an HF. Es wurde gefunden, daß das HF-Addukt durch Behandlung mit HF, gegebenenfalls bei erhöhter Temperatur (80 bis 120 °C) und erhöhtem Druck (z. B. autogener Druck im Autoklaven) regeneriert werden kann. Dabei zeigte es sich, daß es nicht notwendig ist, vorhandenes HCl vollständig auszutreiben. Ein Restgehalt von z. B. weniger als 5 Gew.-%, vorzugsweise weniger als 2 Gew.-% HCl ist akzeptabel.

Gemäß einer Ausführungsform der Erfindung stellt man Sulfurylfluorid als Säurefluorid aus Sulfurylchlorid oder Schwefeldioxid und Chlor (dann unter Druck zwecks Verflüssigung) her. Hier setzt man - zur Freisetzung von HCl - eine Säure zu, beispielsweise eine halogenierte Carbonsäure wie Trifluoressigsäure. Es kann, zwecks (Teil)Neutralisierung dieser zugesetzten Säure, eine Stickstoffbase zugesetzt werden. Dabei entspricht zweckmäßig diese Base der im Hydrofluorid enthaltenen Base.

Sulfurylfluorid kann auch aus Sulfurylchloridfluorid hergestellt werden. Es ist somit eine zweistufige Herstellung von Sulfurylfluorid möglich. Die erste Stufe umfaßt die Herstellung von Sulfurylchloridfluorid aus Sulfurylchlorid. Das Verhältnis von Amin zu HF in der Reaktionsmischung ist bei dieser Stufe nicht begrenzt; die Durchführung gelingt auch mit sehr hohem HF-Gehalt, z. B. bei einem Verhältnis von Amin:HF von 1:3 bis hin zu 1:10 oder auch darüber. Die zweite Stufe, die Fluorierung von Sulfurylchloridfluorid zu Sulfurylfluorid, verlangt ein Verhältnis von Amin zu HF, das größer ist als 1:3; beispielsweise liegt es zwischen 1:2 und 1:3. Diese Maßgabe bezüglich des maximalen Gehalts an HF in der Reaktionsmischung gilt auch bei der einstufigen Herstellung von SO₂F₂ aus SO₂Cl₂, wenn eine gute Ausbeute an SO₂F₂ erzielt werden soll. Es wird angenommen, daß bei einem Verhältnis von Amin:HF unterhalb von 1:3 sich die Reaktivität (Nucleophilie) des F⁻-Anions ändert.

Die zweistufige Herstellung von Sulfurylfluorid ermöglicht es, eine besondere Verfahrensvariante anzuwenden. Es wurde nämlich festgestellt, daß während der Fluorierung von SO₂Cl₂ zu SO₂ClF gleichzeitig die Regenerierung des HF-Adduktes (wie oben erwähnt) möglich ist. Man bringt SO₂Cl₂, einen Überschuß HF und zu regenerierendes HF-Addukt in einen Reaktor ein. Unter erhöhtem Druck (z. B. autogener Druck im Autoklaven) wird simultan SO₂ClF und regeneriertes HF-Addukt erzeugt. Gasförmiges HCl, das sich bildet, wird abgetrennt (z. B. durch Ablassen des Überdrucks und Durchleiten von Inertgas wie N₂). Dann wird HF abgedampft, um den HF-Gehalt auf die obenbeschriebene Limitierung zu bringen (Amin:HF >1:3). Dann kann die zweite Stufe zwecks Herstellung von SO₂F₂ durchgeführt werden. Werden die erste und zweite Stufe im gleichen Reaktor durchgeführt, ist es so möglich, in die zweite Stufe stets mit refluoriertem HF-Addukt zu gehen.

Den Chlor-Fluor-Austausch gemäß der vorliegenden Erfindung führt man vorzugsweise bei einer Temperatur von Umgebungstemperatur (etwa 20 °C) bis 150 °C durch. Bevorzugt liegt das Molverhältnis des HF-Adduktes des Hydrofluorides, bezogen auf die darin enthaltene Base, zu Säurechlorid im Bereich von 1:0,01 bis 1:1 (1 Mol R₃N·2,6 HF und 1 Mol Säurechlorid haben dann ein Verhältnis von 1:1), wenn pro Säurechlorid-Addukt ein Cl-Atom ausgetauscht werden soll. Bei mehreren Chlor-Atomen, die pro Molekül ausgetauscht werden sollen, ist der Einsatz des Hydrofluorids zweckmäßig doppelt, dreifach etc. so hoch. Bei kontinuierlicher Verfahrensweise kann das Verhältnis im Bereich von 1:0,01 bis 1:100 liegen.

Bei der Umsetzung wird bei der Herstellung von Carbonsäurefluoriden spontan Chlorwasserstoff freigesetzt. Dieser Chlorwasserstoff kann aus dem Reaktor abgelassen werden (beispielsweise durch ein entsprechend eingestelltes Überdruckventil). Bei der Herstellung von SO₂F₂ ist zur HCl-Freisetzung die Zugabe von Säuren wie Trifluoressigsäure erforderlich.

Es wurde bei Versuchen festgestellt, daß oftmals ein Zusatz von z. B. Trifluoressigsäure gleich bei Beginn der Fluorierungsreaktion insofern vorteilhaft ist, als das Ausfallen von Feststoffen (die sich später wieder auflösen) verhindert oder reduziert wird. Beispielsweise reichen schon 10 Mol-% der Säure, bezogen auf das als 100 Mol-% berechnete Onium-HF-Addukt, aus.

Es wurde auch festgestellt, daß Ammoniumsalze mit drei C1- oder C2-Alkylresten gebildetes HCl sehr leicht freisetzen; sie neigen allerdings zur Bildung von Feststoffen, so daß der Zusatz von z. B. Trifluoressigsäure, wie vorstehend beschrieben, vorteilhaft ist. Oniumsalze mit drei C3- oder höherkettigen Alkylresten werden zwar nicht fest; aber aus ihnen wird HCl nicht so leicht freigesetzt wie aus den kürzerkettig substituierten Oniumsalzen. Hier ist der Säurezusatz vorteilhaft, weil dadurch verstärkt HCl ausgetrieben wird.

Gemäß der Erfindung fungiert das Hydrofluorid-Addukt als Fluorierungsmittel. Es wird in einer solchen Menge eingesetzt, daß es nicht so weit dehydrofluoriert wird, daß die Stufe des Oniummonohydrofluorids überschritten wird. Wenn beispielsweise ein Addukt der Formel R₃N·2,6 HF eingesetzt wird, soll nur soviel HF verbraucht werden, daß R₃N·zHF mit z=1 oder z>1 im Reaktionsgemisch verbleibt. Das entsprechende Hydrochlorid soll nicht entstehen, wenn man möglichst lange Zeit eine Regenerierung unter Druck mit HF vermeiden will. Es genügt hier, lediglich HF zuzusetzen.

Wenn beispielsweise bei einer Verbindung ein Chloratom gegen ein Fluoratom ausgetauscht werden soll, setzt man pro 1,6 Mol der Ausgangsverbindung mindestens 1 Mol des Adduktes R₃N·2,6 HF ein. Setzt man andere Edukte (z. B. SO₂Cl₂) oder Oniumsalze mit anderem HF-Gehalt ein, so ist die Stöchiometrie entsprechend anzupassen.

Somit weist das Verfahren gemäß der Erfindung den Vorteil auf, daß die Aufarbeitung sehr viel einfacher ist: es fällt kein Aminhydrochlorid als Abfallprodukt an; man muß nicht von einem Lösemittel abtrennen.

Ein weiterer Gegenstand der Erfindung ist eine Zusammensetzung, die fluorierend wirkt. Sie ist erhältlich durch Vermischen von HF-Addukten der Formel B¹·mHF und einer Säure. Diese wirkt derart, daß HCl aus der Reaktionsmischung bei der SO₂F₂-Herstellung freigesetzt wird. Gegebenenfalls kann noch Base B¹ zugesetzt sein, in einer Menge, die geringer ist als die zur Neutralisation der Säure.notwendige Menge. B¹ bedeutet NH₃ oder die Base B, wie oben definiert, m bedeutet 1<m<4. Bevorzugte Säure ist eine halogenierte Carbonsäure wie Trifluoressigsäure. Das HF-Addukt von B¹ kann auch in situ erzeugt werden. Gewünschtenfalls kann man diese Säure oder einen Teil der Säure in Form des Salzes mit B¹ einbringen. Die bevorzugte Zusammensetzung hat die "Formel" B¹·(0,1-1,0)TFA·(1,0-3,0)HF. TFA ist Trifluoressigsäure. B¹ ist bevorzugt B. Bevorzugtes B ist weiter oben angegeben.

Die Verwendung von HF-Addukten von Oniumsalzen des Stickstoffes als Fluorierungskatalysator beim Fluor-Chlor-Austausch und Fluor-Brom-Austausch bei durch weitere elektronegative Substituenten aktivierten Kohlenstoffatomen, besonders bei C(O)Cl- und C(O)Br-Gruppen ist moglich.

Die folgenden Beispiele sollen die Erfindung weiter erläutern, ohne sie in ihrem Umfang einzuschränken. Die Beispiele 1 bis 4 erläutern die Verwendung von HF-Addukten als Fluorierungsmittel (vermutlich autokatalytisch), die Beispiele 5 bis 6 die Verwendung als Katalysator.

### Beispiele

### Beispiele zur Herstellung von SO₂F₂ aus SO₂Cl₂ unter Verwendung von Tributylamin·2,6 HF und Tributylamin·TFA·2,6 HF

SO₂Cl₂ + 2 (CH₃-CH₂-CH₂-CH₂)₃N·2,6 HF + TFA → SO₂F₂ + 2 HCl

### Beispiel 1:

### nachträgliche Zugabe von TFA zur Freisetzung der HCl

### Ansatz:

| | |
|---|---|
| 0,30 Mol Sulfurylchlorid (SO₂Cl₂) | 40, 5 g |
| 0,375 Mol Tributylamin·2,6 HF | 71,2 g |
| 0,375 Mol Trifluoressigsäure | 42,8 g |

### Aufbau und Durchführung aus diesem Beispiel gilt auch für alle nachfolgende Beispiele:

In einem 250 ml Dreihalskolben mit Rückflußkühler, Thermofühler und Topftrichter wurde der Tributylamin-Komplex vorgelegt. Der Rückflußkühler wurde über einen Kryomaten mit -30 °C kalter Sole gespeist. Ein nach dem Rückflußkühler installierter Blasenzähler zeigte das Verlassen von Produkten über den Rückflußkühler an. Um das Reaktionsprodukt aufzufangen, war nach dem Kühler ein Stahlzylinder (mit ca. 300 ml Volumen) mit Tauchrohr und Gasausgang geschaltet, der in einem Dewar mit CO₂ /Methanol auf -78 °C temperiert wurde. Bei Raumtemperatur wurde langsam und unter starkem Rühren SO₂Cl₂ in die Lösung getropft. Die Reaktion war leicht exotherm. Kurze Zeit nach Beginn des Zutropfens war eine Gasentwicklung zu beobachten, die GC-Analyse des Gases nach dem Rückflußkühler zeigte 85,6 % SO₂F₂ neben 14,3 % SO₂FCl (keine HCl) an. Zu Beginn der Reaktion färbte sich die Lösung im Kolben etwas dunkler, wurde jedoch nach einiger Zeit wieder hell, fast farblos.

Da bisher noch keine HCl im Abgas detektiert wurde (notwendig im Einsatz für katalytische Fluorierung) wurde nach Beendigung der Gasentwicklung (Beobachtung Blasenzähler) aus obiger Reaktion vorsichtig Trifluoressigsäure (TFA) zugetropft. Sofort wurde HCl und auch noch weitere Mengen SO₂F₂ aus der Reaktionslösung freigesetzt. Um das Austreiben von Reaktionsprodukten und die Reaktion zu vervollständigen, wurde die Reaktionslösung auf 50 °C temperiert bis am Blasenzähler keine Gasentwicklung mehr zu beobachten war. Die isolierte Ausbeute im Zylinder betrug wegen der unzureichenden Kühlung nur 48 %.

### Beispiel 2:

Versuch mit einem TFA/NBu₃-Verhältnis von 1:1/Sofortiger Einsatz von Bu₃N/HF/TFA als Katalysatormischung

### Ansatz:

| | |
|---|---|
| 0,23 Mol SO₂Cl₂ | 31,7 g |
| 0,20 Mol Tributylamin·2,6 HF | 47,5 g |
| 0,123 Mol Tributylamin | 22,8 g |
| 0,323 Mol Trifluoressigsäure | 36,8 g |

### Aufbau:

Wie bei Beispiel 1.

### Herstellung der fluorierenden Zusammensetzung:

Das HF-Addukt des Tributylamins und das Tributylamin wurden miteinander vermischt. Dann wurde langsam die Trifluoressigsäure zugetropft.

### Durchführung der Fluorierung:

Das SO₂Cl₂ wurde langsam zugetropft. Im Abgas konnte sofort HCl sowie SO₂F₂ und SO₂FCl nachgewiesen werden.

### Beispiel 3:

Versuch mit NBu₃/TFA = 1:0,1

### Ansatz:

| | |
|---|---|
| 0,35 Mol SO₂Cl₂ | 47,2 g |
| 0,30 Mol Tributylamin·2,6 HF | 71,2 g |
| 0,03 Mol Tributylamin | 5,6 g |
| 0,03 Mol Trifluoressigsäure | 3,4 g |

### Aufbau und Durchführung:

Wie bei Beispiel 2.

Das Abgas hatte beim ersten Zutropfen von SO₂Cl₂ eine Zusammensetzung von 73,4 % SO₂F₂ und 25,1 % SO₂FCl und anfangs noch keine HCl. Später veränderte sich die Zusammensetzung etwas zu Gunsten des Sulfurylfluorides auf 97,2 % SO₂F₂ und nur noch 2,0 % SO₂FCl und 0,3 % HCl. Eine Massenbilanz wurde nicht erstellt.

### Beispiel 4:

Versuch mit NBu₃/TFA = 1 : 0,23

### Ansatz:

| | |
|---|---|
| 0,35 Mol SO₂Cl₂ | 47,2 g |
| 0,30 Mol Tributylamin·2,6 HF | 71,2 g |
| 0,09 Mol Tributylamin | 16,7 g |
| 0,09 Mol Trifluoressigsäure | 10,34 g |

### Aufbau und Durchführung:

Wie bei Beispiel 2. In diesem Experiment wurde ein geeigneter Stahlzylinder mit flüssigem Stickstoff gekühlt, um eine Massenbilanz zu erhalten.

Das Abgas hatte zu Beginn des SO₂Cl₂-Zutropfens eine Zusammensetzung von 85,1 % SO₂F₂ und 14,8 % SO₂FCl und 0,6 % HCl. Die im Stahlzylinder einkondensierte Mischung bestand aus 82 % SO₂F₂ und 18 % SO₂FCl und Spuren von HCl und SO₂. Das entspricht einer theoretischen Ausbeute von 90,3 % bezogen auf isolierte Mengen an SO₂F₂ und SO₂FCl.

### Beispiele 5 und 6:

Versuche zur Verwendung von NBu₃·2,6 HF als Fluorierungskatalysator

### Beispiel 5:

Kontinuierliches Verfahren unter Verwendung von Tributylamin·2,6 HF (ohne TFA) als Katalysator

### Ansatz:

| | |
|---|---|
| 0,74 Mol Sulfurylchlorid (SO₂Cl₂) | 100,00 g |
| 0,40 Mol Tributylamin·2,6 HF 0,46 Mol 1,1,1,3,3-Pentafluorbutan | 94,9 g |
| (S 365mfc) | 68,3 g |
| 1,48 Mol HF | 29,6 g |

### Aufbau und Durchführung (gilt für das nachfolgende Beispiel):

In einer 250 ml PFA Waschflasche wurden 365 und Sulfurylchlorid vorgelegt und mit Eis gekühlt. Dann wurde vorsichtig unter Rühren HF eingeleitet. In einem 250 ml Dreihalskolben mit Rückflußkühler, Thermofühler und Topftrichter wurde der Tributylaminkomplex vorgelegt. Der Rückflußkühler wurde über einen Kryomaten mit -30 °C kalter Sole gespeist. Bei Raumtemperatur wurde langsam und unter starkem Rühren die Mischung aus HF/S365/SO₂Cl₂ in die ölige, hellbraune Lösung getropft. Die Reaktion ist exotherm (ΔT = 22 °K), die Innentemperatur stieg bis auf die Siedetemperatur des 365mfc an (41 °C). Das 365mfc wurde durch den Kondensator im Reaktionskolben zurückgehalten. Kurze Zeit nach Beginn des Zutropfens ist eine Gasentwicklung zu beobachten; neben HCl wurde SO₂F₂ und hauptsächlich SO₂FCl freigesetzt. Eine Massenbilanz wurde nicht erstellt.

### Beispiel 6:

Kontinuierliches Verfahren unter Verwendung von Tributylamin·2,6 HF mit TFA als Katalysator

### Ansatz:

| | |
|---|---|
| 0,80 Mol Sulfurylchlorid (SO₂Cl₂) | 107,98 g |
| 0,20 Mol Tributylamin·2,6 HF | 47,74 g |
| 1,60 Mol HF | 32,0 g |
| 0,77 Mol S 365mfc | 113,3 g |
| 0,02 Mol Tributylamin | 3,7 g |
| 0,02 Mol Trifluoressigsäure | 2,3 g |

### Aufbau und Durchführung:

Siehe oben.

Kurze Zeit nach Beginn des Zutropfens war eine Gasentwicklung zu beobachten; neben HCl wurde SO₂F₂ und hauptsächlich SO₂FCl freigesetzt. Das 365mfc wurde durch den Kondensator weitgehendst im Reaktionskolben zurückgehalten. Die HCl-Entwicklung erfolgte etwas früher als im vorherigen Versuch. Eine Massenbilanz wurde nicht erstellt.

### Beispiel 7:

Verwendung von NEt₃·2,6 HF als Fluorierungskatalysator zur Herstellung von SO₂F₂

NEt₃·2,6 HF + SO₂Cl₂ → SO₂F₂

### Ansatz:

| **Stoff** | **Molmasse** | **Masse in g** | **Mol** |
|---|---|---|---|
| Triethylamin·2,6 HF | 153,22 | 58,2 | 0,38 |
| Sulfurylchlorid | 134,97 | 41,6 | 0,31 |

### Durchführung:

In einem Dreihalskolben mit Glaskühler (~0 °C) und abschließendem Blasenzähler wurde Triethylamin·2,6 HF vorgelegt und unter Rühren SO₂Cl₂ bei Raumtemperatur zugetropft. Zu Beginn des SO₂Cl₂-Zutropfens hatte das den Kühler verlassende Abgas eine Zusammensetzung von 63 % SO₂F₂ neben 37 % SO₂FCl. Gegen Ende der Reaktion fiel der SO₂F₂-Gehalt bis auf 56 % SO₂F₂ ab. Eine Massenbilanz wurde nicht erstellt.

### Beispiel 8:

Herstellung von SO₂FCl aus SO₂Cl₂ unter Verwendung von Tributylamin·4,5 HF

SO₂Cl₂ + (CH₃-CH₂-CH₂-CH₂)₃N·4,5 HF → SO₂FCl + HCl

### Ansatz:

| | |
|---|---|
| 0,24 Mol Sulfurylchlorid (SO₂Cl₂) | 47,2 g |
| 0,24 Mol Tributylamin·4,5 HF | 68,4 g |

### Aufbau und Durchführung:

In einem 250 ml Dreihalskolben mit Rückflußkühler mit Blasenzähler, Thermofühler und Tropftrichter wurde das Tributylamin vorgelegt. Der Rückflußkühler wurde über einen Kryomaten mit ca. 0 °C kalter Sole gespeist. Bei Raumtemperatur wurde langsam und unter starkem Rühren SO₂Cl₂ in die ölige, hellbraune Lösung zugetropft. Die Reaktion war leicht exotherm (ΔT = 10 °K). Kurze Zeit nach Beginn des Zutropfens war eine Gasentwicklung zu beobachten. Als Reaktionskontrolle wurden während des Zutropfens GC-Proben aus dem Abgasstrom nach dem Blasenzähler genommen. Zu Beginn der Zugabe von SO₂Cl₂ betrug die Abgaszusammensetzung 55,2 % HCl, neben 42,07 % SO₂FCl; auch SO₂ wurde detektiert. Bei Beendigung des Zutropfens von SO₂Cl₂ war die Abgaszusammensetzung nahezu identisch. Eine Massenbilanz wurde nicht erstellt.

Die Beispiele 10 bis 16 erläutern die Regenerierung ("Recycling") des durch HCl kontaminierten HF-Adduktes.

### Beispiel 9:

### Recycling von NBu₃·1,7 HCl zu NBu₃·Y HF unter Einsatz von wenig HF

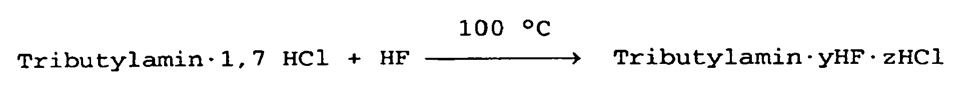

### Ansatz:

| **Stoff** | **Molmasse** | **Masse in g** | **Mol** |
|---|---|---|---|
| Tributylamin·1,7 HCI | 247,34 | 82,17 | 0,33 |
| HF | 20,01 | 50 | 2,5 |

In einem Laborautoklav wurde Tributylamin·1,7 HCl vorgelegt. Nach dem Verschließen wurde 50 g HF zugegeben und ca. 3 h bei 100 °C Reaktorinnentemperatur gekocht. Der Autoklav wurde dann auf ca. 60 °C Reaktorinnentemperatur abgekühlt und die Gasphase bis zum Erreichen von Atmosphärendruck im Autoklaven in eine Waschflasche mit Wasser geleitet.

Bestimmt nach der Chlorid- und Fluoridanalyse der Waschflasche hatte der im Autoklaven verbliebene Katalysator nun eine Zusammensetzung von Tributylamin·0,62 HCl·7,3 HF.

Das Beispiel zeigt, daß selbst vollständig unter Bildung von Hydrochlorid verbrauchtes HF-Addukt regeneriert werden kann. Das regenerierte Addukt konnte wieder in Fluorierungsreaktionen eingesetzt werden.

Das folgende Beispiel zeigt, daß der HCl-Gehalt dieses Produktes noch weiter abgesenkt werden kann.

### Beispiel 10:

Weitere Verringerung des HCl-Gehaltes in NBu₃·0,62 HCl·7,3 HF zu NBu₃·Y HF unter Einsatz eines Überschusses an HF

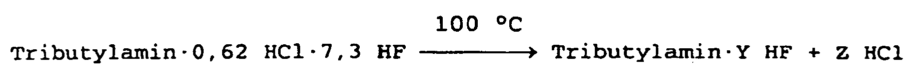

### Ansatz:

| **Stoff** | **Molmasse** | **Masse in g** | **Mol** |
|---|---|---|---|
| Tributylamin·0.62 HCl·7,3 HF | 354,3 | 116,83 | 0,33 |
| HF | 20,01 | 107 | 5,35 |

Zur im Autoklaven verbliebenen Mischung aus Beispiel 9 von Tributylamin·0,62 HCl·7,3 HF wurden erneut nun 107 g HF gegeben und bei ca. 100 °C nun über Nacht gekocht. Der Autoklav wurde dann auf ca. 60 °C Reaktorinnentemperatur abgekühlt und die Gasphase bis zum Erreichen von Atmosphärendruck in eine Waschflasche mit Wasser geleitet. Berechnet anhand der Chlorid- und Fluoridanalyse der Waschflasche hatte der im Autoklaven verbliebene Katalysator nun eine Zusammensetzung von Tributylamin·0,005 HCl·4,89 HF. Diese Zusammensetzung wurden durch Direktanalyse des verbliebenen Rückstandes im Autoklaven bestätigt. Die HCl war somit nahezu vollständig ausgetrieben worden. Zersetzungsprodukte des Amins wurden nicht gefunden.

Die erhaltene Zusammensetzung war hervorragend brauchbar als Fluorierungsreagenz und Fluorierungskatalysator.

### Beispiel 11:

Recycling von NEt₃·1,0 HCl zu NEt₃·Y HF unter Einsatz eines Überschusses an HF

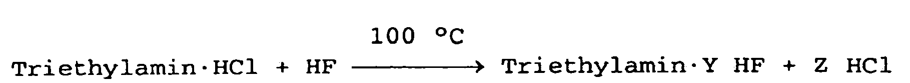

### Ansatz:

| **Stoff** | **Molmasse** | **Masse in g** | **Mol** |
|---|---|---|---|
| Tributylamin·HCl | 137,65 | 37,93 | 0,28 |
| HF | 20,01 | 107,7 | 5,38 |

### Durchführung:

In einem Laborautoklav wurde Triethylamin Hydrochlorid vorgelegt und verschlossen. Danach wurde HF zu gegeben und über Nacht bei 100 °C Reaktorinnentemperatur gekocht. Anschließend wurde bei 100 °C Reaktortemperatur die Gasphase bis zu Atmosphärendruck in eine mit Wasser gefüllte Waschflasche abgelassen. Berechnet anhand der Chlorid- und Fluoridanalyse der Waschflasche hatte der im Autoklaven verbliebene Katalysator nun eine Zusammensetzung von Triethylamin·0,09 HCl·5,35 HF. Diese Zusammensetzung wurde durch Direktanalyse des verbliebenen Rückstandes im Autoklaven bestätigt. Die HCl war somit nahezu vollständig ausgetrieben worden. Zersetzungsprodukte des Amins wurde nicht gefunden.

### Beispiel 12:

Einstellung des Verhältnisses von Amin zu HF auf 2,8 bei der Katalysatormischung aus Beispiel 11

### Durchführung:

Die in Versuch 11 erhaltene Mischung Triethylamin·0,09 HCl· 5,35 HF wurde in eine PFA-Flasche mit Fritte gefüllt und mit trockenem Stickstoff die überschüssige HF ausgetrieben. Nachdem das Gewicht der Flasche 30 min konstant geblieben ist, wurde noch 10 min ein Vakuum von 10⁻³ mbar angelegt um wirklich alle Restmengen HF zu entfernen. Der so erhaltene Katalysator hatte nach Chlorid-, Fluorid- und Aminanalyse nun eine Zusammensetzung von Triethylamin·2,8 HF. HCl wurde nicht mehr gefunden. Die Recycelbarkeit des Katalysators wurde hiermit bewiesen.
Durch die vorgenommene Einstellung des Amin-HF-Verhältnisses werden die nucleophilen Eigenschaften des Adduktes wiederhergestellt. Es eignet sich jetzt hervorragend als Reagenz zur Herstellung von SO₂F₂ aus SO₂Cl₂.

### Beispiel 13 und 14:

Variation der Zeitdauer der Regenerierung

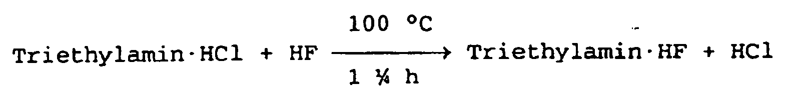

### Ansatz:

| **Stoff** | **Molmasse** | **Masse in g** | **Mol** |
|---|---|---|---|
| Tributylamin·HCl | 137,65 | 10,00 | 0,07 |
| HF | 20,01 | 16,5 | 0,82 |

### Durchführung:

In einem Laborautoklav wurde Triethylamin-Hydrochlorid vorgelegt und verschlossen. Danach wurde HF zu gegeben und 1 ¼ Stunden bei ca. 100 °C Reaktortemperatur gekocht (Autoklav wurde 15 min. vorgeheizt). Nach dieser Zeit wurde die Gasphase innerhalb von 15 min., während der Autoklav im Ölbad stand, abgelassen und analysiert (Probe 1). Der Reaktorinhalt (18,07 g) wurde in eine PFA-Flasche gegeben und 5 min. mit Stickstoff gespült (18,02 g), aus dieser Lösung wurden 1,54 g entnommen und auf 1 1 mit dest. Wasser aufgefüllt und naßchemisch analysiert (Probe 2).

| | **CI [g/l]** | **F [g/l]** | **TEA [g/l]** |
|---|---|---|---|
| **Probe 1** | 1,80 | 8,49 | 0,07 |
| **Probe 2** | 0,02 | 0,80 | 0,74 |

| | | | |
|---|---|---|---|
| TEA = Triethylamin | | | |

Aus den Analysendaten ergibt sich nun eine Katalysatorzusammensetzung von: **NEt₃·5,75 HF·0,08 HCl**

### Beispiel 15:

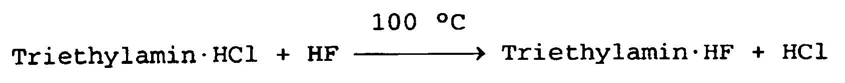

### Ansatz:

| **Stoff** | **Molmasse** | **Masse in g** | **Mol** |
|---|---|---|---|
| Tributylamin·HCl | 137,65 | 10,44 | 0,08 |
| HF | 20,01 | 21,3 | 1,06 |

### Durchführung:

In einem Laborautoklav wurde Triethylamin-Hydrochlorid vorgelegt und verschlossen. Danach wurde HF zu gegeben und 30 min. bei ca. 100 °C Reaktortemperatur gekocht (Autoklav wurde 15 min. vorgeheizt). Nach dieser Zeit wurde der Autoklav aus dem Ölbad genommen und die Gasphase innerhalb von 15 min. abgelassen und analysiert (Probe 1). Der Reaktorinhalt (20,96 g) wurde in eine PFA-Flasche gegeben und 5 min. mit Stickstoff gespült(20,64 g), aus dieser Lösung wurden 1,1 g entnommen und auf 1 1 mit dest. Wasser aufgefüllt und ionenchromatografisch analysiert (Probe 2).

| | **CI [g/l]** | **F [g/l]** | **TEA [g/l]** |
|---|---|---|---|
| **Probe 1** | 2,66 | 1,89 | <0,10 |
| **Probe 2** | 0,02 | 0,67 | 0,42 |

Aus den Analysendaten ergibt sich eine Katalysatorzusammensetzung von : **Triethylamin·8,48 HF**·**0,14 HCl.**

## Patentansprüche

1. Verfahren zur Herstellung von Sulfurylfluorid durch Kontaktieren von Säurechlorid und Fluorwasserstoff-Addukten von Hydrofluoriden organischer Stickstoffbasen ohne Zusatz einer HF-bindenden Base oder eines HF-bindenden Lösemittels, wobei das Fluorwasserstoff-Addukt als Fluorierungsreagenz dient und nicht über die Stufe des Hydrofluorids der organischen Stickstoffbase hinaus dehydrofluoriert wird.

2. Verfahren nach Anspruch 1, **dadurch gekennzeichnet, daß** es kontinuierlich durchgeführt wird.

3. Verfahren nach Anspruch 1, **dadurch gekennzeichnet, daß** man Sulfurylfluorid aus Sulfurylchlorid oder einem Gemisch von Schwefeldioxid und Chlor herstellt, wobei das Molverhältnis von Amin und HF im Reaktionsgemisch oberhalb von 1:3 gehalten wird.

4. Verfahren nach Anspruch 1 zur Herstellung von Sulfurylfluorid unter Regeneration des HF-Adduktes, wobei man in einer ersten Stufe Sulfurylchlorid und HF miteinander umsetzt in Anwesenheit des zu regenerierenden HF-Adduktes, wobei Sulfurylchloridfluorid und regeneriertes HF-Addukt entsteht, und man in einer zweiten Stufe das gebildete Sulfurylchlorid-fluorid zu Sulfurylfluorid umsetzt, in Anwesenheit von HF-Addukt, mit der Maßgabe, daß in der zweiten Stufe das MolVerhältnis von Amin zu HF in der Reaktionsmischung oberhalb von 1:3 gehalten wird.

5. Verfahren nach Anspruch 1, **dadurch gekennzeichnet, daß** man das HF-Addukt eines primären, sekundären oder tertiären aliphatischen Aminhydrofluorids mit bis zu 15 C-Atomen oder eines primären, sekundären oder tertiären Aminhydrofluorids mit mindestens einem aromatischen Rest einsetzt.

6. Verfahren nach Anspruch 5, **dadurch gekennzeichnet, daß** man das HF-Addukt eines Hydrofluorids eines sekundären oder tertiären aliphatischen Amins mit insgesamt bis zu 15 C-Atomen oder eines sekundären oder tertiären Amins mit einer Phenylgruppe einsetzt.

7. Verfahren nach Anspruch 6, **dadurch gekennzeichnet, daß** man das HF-Addukt von Triethylaminhydrofluorid, Tri-n-propylaminhydrofluorid, Tri-iso-propylaminhydrofluorid, Tri-n-butylaminhydrofluorid, Pyridinhydrofluorid, Piperidinhydrofluorid oder N,N-Dimethylaminhydrofluorid einsetzt.

8. Verfahren nach Anspruch 1, **dadurch gekennzeichnet, daß** man bei einer Temperatur von Umgebungstemperatur bis 150 °C arbeitet.

9. Verfahren nach Anspruch 1, **dadurch gekennzeichnet, daß** man eine Säure zusetzt.

10. Verfahren nach Anspruch 9, **dadurch gekennzeichnet, daß** man eine Halogencarbonsäure zusetzt, vorzugsweise Trifluoressigsäure.

11. Fluorierend wirkende Zusammensetzung, erhältlich durch Vermischen von HF-Addukten der Formel B·mHF, worin B sekundäres oder tertiäres Amin ist; 1<m<4; sowie einer halogensubstituierten Carbonsäure.

12. Zusammensetzung nach Anspruch 11 der Formel B¹·(0,1-1,0)TFA·(1,0-3,0)HF, worin B für sekundäres oder tertiäres Amin und TFA für Trifluoressigsäure steht.

13. Zusammensetzung nach Anspruch 12, die zugesetztes B enthält, in einer Menge, die gleich oder geringer ist als die zur Neutralisation der Säure notwendige Menge.

## Claims

1. A method for producing sulphuryl fluoride by contacting acid chloride and hydrogen fluoride adducts of hydrofluorides of organic nitrogen bases without the addition of an HF-binding base or an HF-binding solvent, wherein the hydrogen fluoride adduct serves as a fluorination reagent and is not dehydrofluorinated beyond the stage of the hydrofluoride of the organic nitrogen base.

2. A method according to Claim 1, **characterised in that** it is performed continuously.

3. A method according to Claim 1, **characterised in that** sulphuryl fluoride is prepared from sulphuryl chloride or a mixture of sulphur dioxide and chlorine, the molar ratio of amine and HF in the reaction mixture being kept above 1:3.

4. A method according to Claim 1 for the preparation of sulphuryl fluoride with regeneration of the HF adduct, wherein in a first stage sulphuryl chloride and HF are reacted together in the presence of the HF adduct which is to be regenerated, with sulphuryl chloride fluoride and regenerated HF adduct being produced, and in a second stage the resulting sulphuryl chloride fluoride is reacted to form sulphuryl fluoride, in the presence of HF adduct, with the proviso that in the second stage the molar ratio of amine to HF in the reaction mixture is kept above 1:3.

5. A method according to Claim 1, **characterised in that** the HF adduct of a primary, secondary or tertiary aliphatic amine hydrofluoride with up to 15 C atoms or of a primary, secondary or tertiary amine hydrofluoride with at least one aromatic radical is used.

6. A method according to Claim 5, **characterised in that** the HF adduct of a hydrofluoride of a secondary or tertiary aliphatic amine having a total of up to 15 C atoms or of a secondary or tertiary amine with a phenyl group is used.

7. A method according to Claim 6, **characterised in that** the HF adduct of triethylamine hydrofluoride, tri-n-propylamine hydrofluoride, tri-iso-propylamine hydrofluoride, tri-n-butylamine hydrofluoride, pyridine hydrofluoride, piperidine hydrofluoride or N,N-dimethylamine hydrofluoride is used.

8. A method according to Claim 1, **characterised in that** it is operated at a temperature from ambient temperature to 150°C.

9. A method according to Claim 1, **characterised in that** an acid is added.

10. A method according to Claim 9, **characterised in that** a halocarboxylic acid, preferably trifluoroacetic acid, is added.

11. A composition having a fluorinating action, obtainable by mixing HF adducts of the formula B·mHF, wherein B is secondary or tertiary amine; 1<m<4; and also a halogen-substituted carboxylic acid.

12. A composition according to Claim 11 of the formula B¹·(0.1-1.0)TFA·(1.0-3.0)HF, wherein B stands for secondary or tertiary amine and TFA stands for trifluoroacetic acid.

13. A composition according to Claim 12, which contains added B in a quantity equal to or less than the quantity necessary to neutralise the acid.

## Revendications

1. Procédé de production de fluorure de sulfuryle par mise en contact de chlorure d'acide et d'adduits d'hydrogène fluoré de fluorhydrates de bases azotées organiques sans ajout d'une base liant HF ou d'un solvant liant HF, dans lequel l'adduit d'hydrogène fluoré sert de réactif de fluoration et n'est pas déshydrofluoré par l'étape du fluorhydrate de la base azotée organique.

2. Procédé selon la revendication 1, **caractérisé en ce qu'**il est exécuté en continu.

3. Procédé selon la revendication 1, **caractérisé en ce que** l'on prépare du fluorure de sulfuryle à partir de chlorure de sulfuryle ou d'un mélange de dioxyde de soufre et de chlore, dans lequel le rapport molaire entre amine et HF dans le mélange réactionnel est maintenu au-delà de 1:3.

4. Procédé selon la revendication 1 pour produire du fluorure de sulfuryle avec régénération de l'adduit HF, dans lequel lors d'une première étape, on mélange ensemble du chlorure de sulfuryle et HF en présence de l'adduit HF à régénérer, dans lequel le chlorure-fluorure de sulfuryle et l'adduit HF régénéré se forment, et dans lequel on convertit, lors d'une seconde étape, le chlorure-fluorure de sulfuryle formé en fluorure de sulfuryle, en présence de l'adduit HF, avec pour condition que dans la seconde étape, le rapport molaire entre amine et HF dans le mélangé réactionnel soit maintenu supérieur à 1:3.

5. Procédé selon la revendication 1, **caractérisé en ce que** l'on utilise l'adduit HF d'un fluorhydrate d'amine aliphatique primaire, secondaire ou tertiaire ayant jusqu'à 15 atomes de C ou d'un fluorhydrate d'amine primaire, secondaire ou tertiaire ayant au moins un radical aromatique.

6. Procédé selon la revendication 5, **caractérisé en ce que** l'on utilise l'adduit HF d'un fluorhydrate d'une amine aliphatique primaire, secondaire ou tertiaire ayant au total jusqu'à 15 atomes de C ou d'une amine secondaire ou tertiaire ayant un groupe phényle.

7. Procédé selon la revendication 6, **caractérisé en ce que** l'on utilise l'adduit HF de fluorhydrate de triéthylamine, de fluorhydrate de tri-n-propylamine, de fluorhydrate de tri-iso-propylamine, de fluorhydrate de tri-n-butylamine, de fluorhydrate de pyridine, de fluorhydrate de pipéridine ou de fluorhydrate de N,N-diméthylamine.

8. Procédé selon la revendication 1, **caractérisé en ce que** l'on travaille à une température comprise entre la température de l'environnement et 150 °C.

9. Procédé selon la revendication 1, **caractérisé en ce que** l'on ajoute un acide.

10. Procédé selon la revendication 9, **caractérisé en ce que** l'on ajoute un acide carboxylique halogéné, de préférence de l'acide trifluoroacétique.

11. Composition agissant par fluoration, pouvant être obtenue par mélange d'adduits HF de formule B • m HF, dans laquelle B est une amine secondaire ou tertiaire ; 1 < m <4 ; et d'un acide carboxylique substitué par un halogène.

12. Composition selon la revendication 11 de formule B¹ • (0,1-1,0)TFA • (1,0-3,0)HF, dans laquelle B est une amine secondaire ou tertiaire et TFA est l'acide trifluoroacétique.

13. Composition selon la revendication 12 qui contient B ajouté, dans une quantité qui est inférieure ou égale à la quantité nécessaire pour neutraliser l'acide.
